Europäisches Patentamt

European Patent Office

Office européen des brevets

(11) Publication number: **0 287 213**
**A2**

# EUROPEAN PATENT APPLICATION

(21) Application number: 88302210.5

(51) Int. Cl.⁴: **A61K 6/08**

(22) Date of filing: 14.03.88

(30) Priority: 17.04.87 US 39287

(43) Date of publication of application:
19.10.88 Bulletin 88/42

(84) Designated Contracting States:
CH DE FR GB IT LI SE

(71) Applicant: **Kerr Manufacturing Corporation**
**28200 Wick Road Box 455**
**Romulus Michigan 48174(US)**

(72) Inventor: **Waller, Duncan Ewan**
**5504 New Meadow Drive**
**Ypsilanti Michigan 48174(US)**

(74) Representative: **Oliver, Roy Edward et al**
**POLLAK MERCER & TENCH High Holborn**
**House 52-54 High Holborn**
**London WC1V 6RY(GB)**

(54) Visible light activated cavity liner.

(57) A visible-light-activated cavity liner, which provides an available source of leachable calcium and fluoride to the base of a tooth cavity, comprises a photopolymerizable matrix material, a photo-initiator, a reducing agent and, as filer material, a synthetic hydroxyapatite and a powdered glass ionomer.

EP 0 287 213 A2

## VISIBLE-LIGHT-ACTIVATED CAVITY LINER

The present invention relates to a light-activated cavity liner for use in the dental field.

Calcium hydroxide has been widely used as the basis for many cavity liners and bases for at least the past four decades and it is commonly believed that the presence of calcium ions stimulates the formation of reparative dentine at the base of tooth cavities, thus aiding the natural healing process. Additionally, these calcium hydroxide-based cavity liners and bases help to protect the pulp of the tooth from any leachable constituent of dental filling materials used to fill the cavity.

In recent years, however, there is increasing evidence which suggests that the prevention of microleakage around a tooth restoration and through the base of a cavity to the pulp is a prime requisite for rapid and successful healing and the prevention of secondary or recurrent caries.

Also during recent years, there has been a large increase in the use of light-curable and, particularly, visible-light-curable dental restorative materials, as the versatility and aesthetic potential of these materials has become apparent and widely recognised.

Very recently, a visible-light-curable cavity base or liner composition based on calcium hydroxide has been commercially developed. Although this material is a single component composition, requiring no mixing, and is curable by the use of a standard composite restorative curing light, there are many ways in which it can be improved, so as to approach more nearly the idealized material indicated by current restorative and histological research. These aspects will be explained in detail in the following summary of the invention, which is based upon discoveries which enable improved light-curable compositions to be provided.

According to the present invention, a visible-light-activated cavity liner comprises a photopolymerizable matrix material, a photo-initiator, a reducing agent and at least one filler and is characterised in that a synthetic hydroxyapatite filler and a powdered glass ionomer filler are included in the composition.

In practice, the main ingredients are usually supplemented by the incorporation of at least one radiopaquing agent, to facilitate radiographic detection of the material in vivo, an adhesion promoter, to enhance adhesion of the composition to tooth structure, at least one pigment, to confer tooth shading on the material, and, optionally, at least one thickener, to reduce any tendency of the paste to undergo separation, during storage prior to use.

A review of current dental restorative and histological research indicates that the following are deemed to be requirements for the idealized cavity base or liner material:

1. Availability of leachable calcium;
2. Availability of leachable fluoride, to minimize the formation of secondary caries;
3. Minimization of microleakage, by providing a protective barrier which is as impervious as possible;
4. Greater radiopacity than tooth structure, for radiographic differentiation;
5. Sufficient hydrophilic properties to wet dentine adequately in vivo;
6. Ability to bond both to dentine and to composite dental restorative materials;
7. Natural dentine appearance;
8. Ability to provide offshade dentine matching;
9. Optimized placement consistency;
10. Ready curability using a standard visible-light curing unit;
11. Extreme resistance to standard acid etchants;
12. Near neutral pH for maximum healing potential;
13. Excellent pulpal histology;
14. Extremely low solubility and disintegration;
15. Non-bioresorbability.

The above and other objectives are accomplished by compositions in accordance with the present invention, as is more fully described in the following detailed description.

The essential components of the visible-light-activated cavity liner of the present invention are a photopolymerizable matrix material, a photo-initiator, a reducing agent, a synthetic hydroxyapatite filler and a powdered glass ionomer filler. More specifically, the various components of the present invention preferably comprise the following:

A low-toxicity acrylic functional resin binder, such as ethoxylated bisphenol-A-dimethacrylate, the adduct of bisphenol-A and glycidyl methacrylate known as BisGMA or, as is preferred, blends of either with bisphenol-A-dimethacrylate.

A hydrophilic comonomer diluent, such as hydroxyethyl methacrylate or hydroxpropyl methacrylate, though other hydroxyl functional comonomers can also be used.

2

Synthetic hydroxyapatite, $3Ca_3(PO_4)_2.Ca(OH)_2$, which is used as a filler containing leachable calcium ions, although calcium hydroxide, calcium oxide or many other calcium-containing compounds or minerals can optionally be substituted for synthetic hydroxyapatite.

Commercially-available glass ionomer powder, as sold for dental use, which is used as a filler containing leachable fluoride ions. Such materials comprise basic fluoro-aluminosilicate glasses, as are available from many sources. One suitable material is available from Espe of Germany under the tradename "Ketac" powder. Another suitable ionomer powder has the following approximate composition by weight, is available form Industrial Corp. of Glenmore, PA and is identified as IG-91-2589:

silicon dioxide        31
aluminium oxide        24
sodium aluminium fluoride        18
aluminium phosphate        15
aluminium fluoride        12

A high level of radiopacity is conferred upon the material by the inclusion of a major proportion of barium sulphate as a third filler component, although other radiopaquing agents, such as barium tungstate or barium aluminium borosilicate, may optionally be substituted. The ability to adhere to a prepared dentine substrate is provided by the inclusion of an organofunctional silane, such as methacryloxypropyltrimethoxysilane, although other materials could also be substituted.

Dentine-like pigmentation is achieved by the incorporation of suitable particulate pigments, such as iron oxides, animal charcoal or other equivalent material.

A combination of a photoreactive diketone and a synergistic tertiary amine reducing agent, preferably acrylic functional, is used as a photosensitizing couplet, although any photosensitizing system which will function effectively when the material is exposed to a high-intensity dental radiation source of about 375-550 nanometre wavelength output may be substituted.

Typical photosensitizers include benzophenone, acetophenone, thioxanthen-9-one, 9-fluorenone, anthraquinone, 4'-methoxyacetophenone, diethoxyacetophenone and the diketones, such as biacetyl, 2,3-pentanedione, benzil, 4,4'oxidibenzil and dl-camphroquinone. Camphroquinone and diketones absorb mostly in the visible light spectrum, between 400 and 500 nanometres. Formulations with these initiators cure readily with visible radiation.

The dental impression materials may also contain a reducing agent, which reduces the ketonic photosensitizers when they are in the excited state and accelerates the rate of polymerization. These materials comprise organic armines, aliphatic or aromatic, monoamines or polyamines, primary, secondary or tertiary. Diethylaminoethyl-methacrylate is a typical material. The tertiary amines are generally preferred. Suitable tertiary amines are described in US-A-3,759,807. Tertiary amines with additional functional groups can also be employed, such as 4,4'bis (dimethylamino)-benzophenone, N-methyldiethanolamine, 4-dimethylaminobenzoate, dimethylaminobenzaldehyde, dimethylaminoethylmethacrylate and dimethylaminoethylacrylate.

The following examples illustrate the broad and preferred concentration ranges for formulations of the present invention. All percentages are by weight.

## EXAMPLE 1

| BROAD FORMULATION | BROAD CONCENTRATION | |
|---|---|---|
| Ethylenically-unsaturated resin | 15 | – 60 |
| Radiopaquing agent | 10 | – 50 |
| Glass ionomer powder | 5 | – 30 |
| Synthetic hydroxyapatite | 5 | – 30 |
| Hydrophilic ethylenically-unsaturated diluent | 2 | – 20 |
| Pigment blend | 0.2 | – 10.0 |
| Polymerizable adhesion promoter | 0.1 | – 2.0 |
| Synergistic amine reducing agent | 0.05 | – 2.0 |
| Visible-light photosensitizer | 0.01 | – 2.0 |

## EXAMPLE 2

| | BROAD CONCENTRATION | | PREFERRED RANGE | |
|---|---|---|---|---|
| Ethoxylated bisphenol-A-dimethacrylate | 15 | – 60 | 25 | – 40 |
| Barium sulphate | 10 | – 50 | 20 | – 25 |
| Glass ionomer powder | 5 | – 30 | 10 | – 20 |
| Synthetic hydroxyapatite | 5 | – 30 | 10 | – 20 |
| Hydroxyethyl methacrylate | 2 | – 20 | 4 | – 10 |
| Yellow iron oxide pigment blend ($Fe_2O_3$) | 0.1 | – 5.0 | 1 | – 3 |
| Grey animal charcoal/$TiO_2$ pigment blend | 0.1 | – 5.0 | 1 | – 3 |
| Methacryloxypropyltrimethoxy-silane | 0.1 | – 5.0 | 0.5 | – 2.0 |
| Tertiary amine reducing /agent | 0.05 | – 2.0 | 0.1 | – 1.0 |
| $\alpha\beta$-Diketone photosensitizer | 0.01 | – 2.0 | 0.05 | – 1.0 |

A specific example of one embodiment of the present invention is as follows:

EXAMPLE 3

| | WT. PERCENT |
|---|---|
| Ethoxylated bisphenol-A-dimethacrylate | 17.0 |
| Bisphenol-A-dimethacrylate | 17.0 |
| Hydroxypropyl-methacrylate | 7.5 |
| Barium-aluminium-borosilicate | 24.0 |
| Synthetic hydroxyapatite | 16.0 |
| Glass ionomer powder | 16.0 |
| Diethylaminoethylmethacrylate | 0.5 |
| Benzil | 0.5 |
| Methacryloxypropyltrimethoxy-silane | 1.0 |
| Pigments | 0.5 |

All of the ingredients which make up compositions of the present invention may be used within the stated preferred ranges, advantageously towards the midpoint of these ranges, in virtually any chosen combination, with a good measure of success, since by their intended end use these materials are only required to be used in relatively thin layers, up to a maximum of about one millimetre thickness, which are readily curable.

The following is a typical method suitable for use in preparing formulations of the present invention, although other known methods of the art may also be used:

In a closed area of filtered light below 550 nanometres wavelength, the following ingredients are blended together in a double planetary mixer in the stated order:

EXAMPLE 4

| | |
|---|---|
| Ethoxylated-bisphenol-A-dimethacrylate (liquid) | 32.0 |
| Hydroxyethyl-methacrylate (liquid) | 6.6 |
| Dimethylaminoethyl-methacrylate (liquid) | 0.55 |
| Methacryloxypropyltrimethoxy-silane (liquid) | 1.0 |
| 2,3-Bornenedione (soluble solid) | 0.45 |
| Yellow Pigment Blend (paste) | 2.0 |
| Grey Pigment Blend (paste) | 2.0 |
| Synthetic hydroxyapatite (solid) | 15.0 |
| * Glass ionomer powder (solid) | 15.0 |
| Barium sulphate (solid) | 22.5 |
| Sub-micron silica (solid) | 3.0 |

The resulting thixotropic paste of 30,000 ± 5% centipoises viscosity is then rollmilled to maximise its homogeneity, followed by packaging in lightproof containers. A sample of the rollmilled paste is tested for performance by placing it in a circular "Teflon" (Regd. Trademark) mould of 6.5 millimetres diameter and 1.5 millimetres depth, laid on a microscope slide backed by white paper. The mould is closed by squeezing out the excess paste with a second microscope slide and the paste is then exposed to a visible light source of approximately 15,000 microwatts per square centimetre intensity, for a time of 20 seconds, at a range or distance of one centimetre. The cured specimen is demoulded, by carefully moving the two microscope slides in opposite directions to break the surface seals, and then tested for hardness with a Barber-Coleman hardness tester. Top/Bottom readings of 90/85 ± 2 should be obtained if the material has been satisfactorily

*IG-91-2589;     Industrial     Corp.;     Glenmore,     PA

formulated.

Physical properties of the actual material used in a monkey histology tooth cavity placement study are as follows, together with comparative test data for the only existing visible-light-curable cavity base or liner composition, based on calcium hydroxide, and data for a typical dental glass ionomer base or liner material:

## TABLE

| | MATERIAL OF INVENTION: EXAMPLE 4 | COMMERCIAL CAVITY LINER MATERIAL* | COMMERCIAL CAVITY LINER MATERIAL** |
|---|---|---|---|
| Form:- | Single Paste | Single Paste | Powder/Liquid |
| Proportioning:- | None | None | 1.3 to 1 by weight |
| Mixing:- | None | None | 40 secs. |
| pH:- | 6.5 | 9.5 | 2.0 (as mixed) |
| Work Time:- | As desired up to 5 min. | Up to 4 min. | 3 min. |
| Set Time:- | 20 seconds | 20 seconds | 6 min. |
| Compressive Strength (24 hrs):- | 29,500 PSI | 13,500 PSI | 18,000 PSI |
| Diametral Tensile Strength (24 hrs):- | 2250 PSI | <2000 PSI | 1960 PSI |
| Water Solubility (7 days) | 0.21% | 0.5% | 0.35% |
| Fluoride Release (21 days) | >2900 Micrograms | NIL | 1800 Micrograms |
| Calcium Content | 5.92% | 8% | 9.44% |
| Form of Calcium:- | Hydroxyapatite | Calcium hydroxide | Basic Glass |
| Calcium Release (7 days):- | 0.206% | >0.2% | None Detectable |
| Radiopacity:- | Good | Fair | Fair |
| Resistance to 37.5% Phosphoric Acid:- | No Change | <0.18% Sol. | Etched |

*Light-cured calcium hydroxide cavity liner sold by Dentsply under the tradename "DYCAL".

**A glass ionomer cavity liner available from GC International (Japan) under the tradename GC GlASS Ionomer Lining Cement.

It will be readily seen from the data in the above TABLE that the material of the present invention has a physiologically neutral pH, superior strength properties when cured and, lower water solubility and it provides a source of leachable calcium and fluoride ions. Further, it has excellent resistance to 37% phosphoric acid, which is customarily used to etch the cavo surface margins of tooth cavities to enhance their bond strength to composite restorative materials, and is truly radiopaque.

Although particular embodiments of the present invention have been disclosed herein for purposes of explanation, further modifications or variations thereof will be apparent to those skilled in the art to which this invention pertains.

## Claims

1. A visible-light-activated cavity liner composition comprising a photopolymerizable matrix material, a photo-initiator, a reducing agent and at least one filler, characterised in that a synthetic hydroxyapatite filler and a powdered glass ionomer filler are included in the composition, whereby the liner composition, when used, provides a source of leachable calcium and fluoride to the base of a tooth cavity.

A visible-light-activated liner composition according to claim 1, which is in the form of a paste and comprises, in weight percent:

Ethylenically-unsaturated resin        15 - 60
Radiopaquing agent        10 - 50
Glass ionomer powder        5 - 30
Synthetic hydroxyapatite        5 -30
Hydrophilic ethylenically-unsaturated diluent        2 - 20
Pigment        0.2 - 10.0
Polymerizable adhesion promoter        0.1 - 2.0
Synergistic amine reducing agent        0.05 - 2.0
Visible-light photosensitizer        0.01 - 2.0

3. A visible-light-activated cavity liner composition according to claim 1 or 2, which comprises in weight percent:

Ethoxylated bisphenol-A-dimethacrylate        15 - 60
Barium sulphate        10 - 50
Glass ionomer powder        5 - 30
Synthetic hydroxyapatite        5 - 30
Hydroxyethyl-methacrylate        2 - 20
Yellow pigment blend        0.1 - 5.0
Grey pigment blend        0.1 - 5.0
Methacryloxypropyltrimethoxy-silane        0.1 - 5.0
Tertiary amine reducing agent        0.05 - 2.0
$\alpha\beta$-Diketone photosensitizer        0.01 - 2.0

4. A visible-light-activated cavity liner composition according to claim 3, which comprises in weight percent:

Ethoxylated bisphenol-A-dimethacrylate        25 - 40
Barium sulphate        20 - 25
Glass ionomer powder        10 - 20
Synthetic hydroxyapatite        10 - 20
Hydroxyethyl-methacrylate        4 - 10
Yellow pigment blend        1 - 3
Methacryloxypropyltrimethoxy-silane        0.5 - 2.0
Tertiary amine reducing agent        0.1 - 1.0
$\alpha\beta$-Diketone photosensitizer        0.05 - 1.0

5. A visible-light-activated liner composition according to any preceding claim, which is in the form of a paste and comprises in weight percent:

Ethoxylated bisphenol-A-dimethacrylate        17.0
Bisphenol-A-dimethacrylate        17.0
Hydroxypropyl-methacrylate        7.5
Barium-aluminium borosilicate        24.0
Synthetic hydroxyapatite        16.0

Glass ionomer powder     16.0
Diethylaminoethylmethacrylate     0.5
Benzil     0.5
Methacryloxypropyltrimethoxy-silane     1.0
Pigments     0.5